Europäisches Patentamt

(19) European Patent Office

Office européen des brevets

(11) Publication number: **0 086 018**
**B1**

(12) EUROPEAN PATENT SPECIFICATION

(45) Date of publication of patent specification: **18.12.85**

(51) Int. Cl.⁴: $C\ 07\ C\ 125/06$

(21) Application number: **83200160.6**

(22) Date of filing: **27.01.83**

(54) Preparing carbamic acid esters.

(30) Priority: **09.02.82 IT 1951982**

(43) Date of publication of application:
**17.08.83 Bulletin 83/33**

(45) Publication of the grant of the patent:
**18.12.85 Bulletin 85/51**

(84) Designated Contracting States:
**AT BE CH DE FR GB LI LU NL SE**

(56) References cited:
**US-A-3 956 360**

(73) Proprietor: **ANIC S.p.A.**
**Via Ruggero Settimo, 55**
**I-90139 Palermo (IT)**

(72) Inventor: **Giroldini, Villiam**
**Via Pace 6**
**I-20097 S. Donato Milanese (Milan) (IT)**

(74) Representative: **Roggero, Sergio et al**
**Ing. Barzanò & Zanardo Milano S.p.A. Via**
**Borgonuovo 10**
**I-20121 Milano (IT)**

## Description

This invention relates to a process for preparing carbamic acid esters by carbonylation of aromatic nitro-compounds with carbon monoxide and an aliphatic alcohol in the presence of a catalyst system composed of a selenium compound and pyridine, characterized in that FeSe or VOSe is used as the selenium compound, and the catalyst system further comprises potassium iodide as a co-catalyst, and in that the carbonylation is carried out by heating to 170°C and under a pressure of from 25 to 30 bars.

The commercial importance of the carbamates is well known, inasmuch that they can profitably be used as plant medicaments and as useful intermediates for the synthesis of aromatic isocyanates.

The prior art points out a number of ways to prepare carbamic acid esters.

One of the oldest methods is to react an isocyante with an alcohol: it is an expensive procedure which employs toxic starting materials, and on the top of that, any one can see that, to produce isocyanates from carbamates would be costly and cumbersome reversal.

The direct carbonylation of nitro-compounds in the presence of at least one alcohol, said reaction being catalyzed by salts, more frequently chlorides, of expensive metals such as rhodium and palladium, optionally in the presence of an organic base, has also been attempted by several Authors and examples of such procedures can be found, for instance, in the GB—1,087,896, the USA—4,178,455 and the BE—837,563 patents. These documents, however, suggest procedures which are impaired by a poor selectivity, the necessity of disposing of undesirable by-products and the requirement of recovering expensive metals.

The reaction of an aromatic amine with an alcohol and carbon oxysulphide in the presence of baryum molybdate had also been attempted, but toxic starting materials had to be used and the results were not particularly brilliant.

A closer approach to the problem in question can be found in the USA—3,956,360 which teaches a carbonylation procedure wherein an alchol and a nitrogenous organic compound are reacted at elevated temperatures and pressures in the presence of a catalyst consisting of an element, of the Group VI A of the Periodic Table, oxygen and polonium excluded, or a compound of an element so defined.

The process according to the USA—3,956,360 however, is impaired by several drawbacks, namely:

1. It requires comparatively high pressure, from 800 to 2600 pounds per square inch (gauge) which correspond to a pressure range of from about 56 bar to about 182 bar.

2. The amounts of selenium compounds, and the like, suggested in the examples of USA—3,956,360 cannot be considered, strictly speaking, catalytic amounts proper: this is, presumably, the reason why the reference patent claims "an active amount", and, quite incidentally, no claim is directed to selenium compounds in the USA patent, but emphasis is given to tellurium compounds, rather.

In order to achieve the twofold objective of working under a compartively low pressure and of using the least possible amounts of metallic compounds having a catalytic activity, the present invention provides a process for preparing carbamic acid esters by reacting an aromatic nitro-compound with carbon monoxide and an aliphatic alcohol, in the presence of a catalyst system composed of at least a selenium compound and a nitrogenous organic base, said process being characterized in that the catalyst system further comprises potassium iodide as a co-catalyst.

In the practical use of the process of this invention, it has also been ascertained that, among the selenium compounds which are a part of the catalyst system, those which have proven to be the most susceptible to having their activity potentiated by potassium iodide, are the metallic selenides, FeSe and VOSe. On the other hand, independently of the use of potassium iodide as a co-catalyst, it has concurrently been ascertained that the metallic selenides aforementioned, and also Tin selenide, show a catalytic activity which is improved over that of the elemental selenium (and the selenium compounds of the prior art as represented by the US 3,956,360 aforesaid.

The process according to the invention will be better illustrated by a few practical examples, numbered from 1 to 8 it being understood that Examples 1 and 2 are comparative examples, whereas Examples 3 to 8 are examples which teach the useful function of potassium iodide, which was wholly unpredictable in view of the prior art. More particularly, on comparing Example 1 with Examples 3, 5 and 7 on the one hand, and Example 2 with Examples 4, 6 and 8, it can be appreciated, at a glance, that the process according to the invention definitely improves both the conversion ratings and the selectivity.

Example 1

A 200-ml autoclave (approximate volume) is charged with 0.5 g (grams) of FeSe, 5 mls of pyridine, 10 mls of nitróbenzene (0.1 mol) and 40 mls of ethyl alcohol.

The autoclave is heated to 170°C with stirring and, as soon as the temperature is stabilized, carbon monoxide is introduced under the pressure of 30 bars. As the pressure drops to 23 bars, the gas is let off to a pressure of 9 bars, whereafter fresh carbon monoxide is introduced under the pressure of 30 bars.

These operations are repeated as necessary.

After 5.5 hours the autoclave is allowed to cool, the gases are bled off and the contents is GLC-analyzed (GLC=Gas Liquid Chromatography).

The results are:

Aniline=0.3 mM (millimols)

Nitrobenzene=45.2 mM

N-Phenyl-ethylcarbamate=51.5 mM

The conversion is 55% and the selectivity is 98%.

The catalyst is filtered off from the raw product of the reaction and the carbamate is purified by fractional distillation.

b.p.=120°C under 6 mmHg (millimeters of mercury)

m.p.=52°C.

Example 2

A 200-ml autoclave is charged with 0.5 of VOSe, 5 mls pyridine, 10 mls nitrobenzene, 40 mls of ethyl alcohol. The autoclave is heated to 170°C with stirring whereafter carbon monoxide is charged under a pressure of 30 bars, the procedure being the same as in Example 1. After 9 hours the autoclave is allowed to cool, vented and the contents is GLC-analyzed. The results are:

Aniline=6.7 mM

Nitrobenzene=43.1 mM

N-phenyl-ethylcarbamate=47.1 mM

The conversion is 57% and the selectivity is 87.5%.

The product is purified as in Example 1.

Example 3

A 200-ml autoclave is charged with 0.5 g of FeSe, 0.61 g of KI, 5 mls of pyridine, 10 mls of nitrobenzene (0.1 mol) and 40 mls of $(CH_3)_2CHOH$. The autoclave is heated to 170°C with stirring and then charged with carbon monoxide under a pressure of 30 bars. After 5 hours the reaction is over. The GLC-analysis gives the following results:

Nitrobenzene=0.0

N-phenyl-isopropylcarbamate=96 mM

The conversion is 100% and the selectivity is 96%.

The raw product, after that the catalyst is filtered off, is evaporated under a reduced pressure to remove pyridine and isopropyl alcohol. A solid crystalline residue is obtained, which is the raw carbamate. The product is purified by cristallization from toluene.

N-phenyl-isopropylcarbamate has a m.p. of 87°C—88°C.

Example 4

A 200-ml autoclave is charged with 0.5 g of VOSe, 0.56 g of KI, 0.3 g of $CH_3COONa$, 5 mls of pyridine, 10 mls of nitrobenzene and 45 mls of ethyl alcohol. The autoclave is heated to a temperature of 170°C with stirring and charged with carbon monoxide under a pressure of 30 bars. The reaction is completed within 2 hours.

N-phenyl-ethylcarbamate is obtained with a selectivity of 88.6% and a conversion of 100%.

The product is purified with the same procedure as in Example 1.

Example 5

A 200-ml autoclave is charged with 0.5 g of FeSe, 0.56 g of KI, 5 mls of pyridine, 40 mls of ethanol, 13.9 of p.nitrophenol (0.1 mol). The autoclave is heated to a temperature of 170°C with stirring and charged with carbon monoxide under a pressure of 30 bars. After 4.5 hours the reaction is over. There are obtained 96 mM of N-p.hydroxyphenul-ethylcarbamate. The conversion is 100% and the selectivity is 96%. The raw product of the reaction is filtered, the solvent evaporated off the product is then isolated by cristallization from toluene.

Example 6

A 200-ml steel autoclave is charged with 0.5 g of VOSe, 0.5 g of KI, 0.3 g of $CH_3COONa$, 5 mls of pyridine, 50 mls of ethanol and 18.2 g of 2,4-binitrotoluene (0.1 mol). The autoclave is heated to the temperature of 150°C, whereafter carbon monoxide is introduced under a pressure of 30 bars. After 4.5 hours the reaction is completed, the raw product of the reaction is filtered and the solvent is evaporated off. The residue is subjected to fractional crystallization from toluene-hexane. By so doing there are isolated 19 g of tolyl-2,4-bis-diethylcarbamate. The conversion is 100% and the selectivity is 72%.

Example 7

A 200-ml autoclave is charged with 0.5 of FeSe, 0.6 of KI, 5 mls of pyridine, 40 mls of ethanol and 15.3 g of p.methoxynitrobenzene (0.1 mol). The autoclave is heated to the temperature of 170°C and then charged with carbon monoxide under a pressure of 30 bars. The reaction is completed after 2.5 hours. In the raw product of the reaction there are determined, by GLC (Gas-Liquid Chromatography) 95 mM of N-P. methoxyphenyl-ethylcarbamate. The conversion is 100% and the selectivity is 95%. The solvent is evaporated off from the raw product of the reaction, whereafter the product is purified by extraction and crystallization with hexane-toluene.

Example 8

A 200-ml autoclave is charged with 0.5 g of VOSe, 0.25 g of $CH_3COONa$, 0.5 g of KI, 5 mls of pyridine, 45 mls of ethyl alcohol and 15.75 g of p.chloronitrobenzene (0.1 mol). The autoclave is heated to 170°C and then charged with carbon monoxide under a pressure of 25 bars. After 7 hours the reaction is over. The GLC-analysis of the raw product of the reaction indicates the occurence of:

N,N-p.dichlorobiphenylurea=2 mM

p.chloronitrobenzene=18 mM

N-p.chlorophenyl-ethylcarbamate=78 mM

The conversion is 78% and the selectivity is 95%.

The solvent is evaporated off from the reaction raw product whereafter the unreacted p.chloronitrobenzene is distilled off under reduced pressures. The residue is fractionally crystallized from hexane thus obtaining the pure carbamate.

**Claim**

A process for preparing esters of carbamic acids by carbonylating aromatic nitro-compounds with carbon monoxide and an aliphatic alcohol in the presence of a catalyst system composed of a selenium compound and pyridine, characterized in that FeSe or VOSe is used as the selenium compound and the catalyst system further comprises potassium iodide as the co-catalyst, and in that the carbonylation is carried out by heating to 170°C and under a pressure of from 25 to 30 bars.

**Patentansprüch**

Verfahren zur Herstellung von Carbaminsäure-estern durch Carbonylieren aromatischer Nitro-verbindungen mit Kohlenmonoxid und einem aliphatischen Alkohol in Gegenwart eines aus einer Selenverbindung und Pyridin zusammengesetzten Katalysatorsystems, dadurch gekennzeichnet, daß FeSe oder VOSe als Selenverbindung verwendet wird und das Katalysatorsystem zusätzlich Kaliumjodid als Co-Katalysator enthält und daß die Carbonylierung durch Erhitzen auf 170°C und unter einem Druck von 25 bis 30 bar ausgeführt wird.

**Revendication**

Procédé pour la fabrication d'esters d'acides carbamiques par carbonylation de composés nitrés aromatiques avec du monoxyde de carbone et un alcool aliphatique en présence d'un système catalyseur constitué d'un composé du sélénium et de pyridine, caractérisé en ce que FeSe ou VOSe est utilisé à titre de composé du sélénium et que le système catalyseur comprend en outre de l'iodure de potassium comme co-catalyseur, et que la carbonylation est effectuée par chauffage à 170°C et sous une pression de 25 à 30 bars.